# EUROPÄISCHE PATENTSCHRIFT

(11) **EP 1 407 727 B1**
(45) Veröffentlichungstag und Bekanntmachung des Hinweises auf die Patenterteilung: **03.08.2005**
(21) Anmeldenummer: 03017531.9
(22) Anmeldetag: 06.08.2003
(51) Int. Cl.: A61F 2/28, A61F 2/78

(54) **Subkutanes, intramuskuläres Lager für ein starres transkutanes Implantat**
Subcutaneous, intra-muscular coupling for a rigid transcutaneous implant
Accouplement intramusculairé sous-cutané pour un implant transcutané fixe

(30) Priorität: 08.10.2002 DE 10247397; 12.03.2003 DE 10311990
(43) Veröffentlichungstag der Anmeldung: 14.04.2004
(73) Patentinhaber: ESKA Implants GmbH & Co., 23556 Lübeck (DE)
(72) Erfinder: Grundei, Hans, Dr. Ing., 23558 Lübeck (DE)
(74) Vertreter: Fuchs Mehler Weiss & Fritzsche

(56) Entgegenhaltungen:
- DE-A- 10 040 590
- DE-C- 19 931 882

## Beschreibung

Die Erfindung betrifft ein subkutanes, intramuskuläres Lager für ein starres transkutanes Implantat, welches intrakorporal in einem Knochenstumpf verankerbar ist und welches ein Zwischenstück zwischen dem intrakorporalen zu verankernden Teil und einer daran ankoppelbaren extrakorporalen Kopplungseinrichtung aufweist.

Ein derartiges Lager ist bekannt aus der DE 100 40 590 A1. Das darin beschriebene Lager besteht aus einem flexiblen Material und es weist eine Tülle auf, die das Implantat distal fest umschließt, sowie eine intrakorporal anzuordnende Überwurfhülse in Form eines flexiblen Faltenbalges, der proximal mit einem angeformten Bund in abdichtender Weise mit der Tülle verbunden ist, derart, dass zwischen der Innenwandung des Faltenbalges und Außenwandung der Tülle ein Hohlraum einer Mindestbreite frei bleibt. Dabei ist distal am Faltenbalg ein flexibles Gitternetzwerk angeordnet, dem sich distalseitig ein weiteres Gitternetzwerk mit einem höheren E-Modul anschließt.

Mit diesem Lager wird das Ziel verfolgt, dass sich Weichteile gegenüber dem starren Implantat bewegen können, ohne dass die Durchbruchstelle im Körperstumpfteil einem erhöhten Risiko einer Entzündung ausgesetzt wird.

Wenn dieses bekannte Lager auch bereits in der Praxis erfolgreich eingesetzt worden ist, birgt es das Risiko, dass im Falle beispielsweise einer Reinigung der Durchtrittstelle des Implantates durch den Oberschenkelstumpf mit einer Kanüle durch das flexible Material, in den meisten Fällen Silikon, hindurch gestochen wird und eine Verkeimung stattfindet.

Vor diesem Hintergrund ist es nun die Aufgabe der vorliegenden Erfindung, ein gattungsgemäßes, subkutanes intramuskuläres Lager der eingangs genannten Art so weiterzubilden, dass die Sicherheit gegen eine Verkeimung der Durchtrittstelle des Implantates und der angrenzenden Bereiche des Oberschenkelstumpfes deutlich erhöht wird und dass ein versehentliches Entfernen der Keimschranke verhindert wird.

Gelöst wird diese Aufgabe durch das Lager mit den Merkmalen des Anspruches 1. Vorteilhafte Weiterbildungen ergeben sich aus den Unteransprüchen.

Demgemäß wird vorgeschlagen, dass das Lager eine mit dem Zwischenstück fest verbundene starre Buchse derart aufweist, dass zwischen der Wandung der Buchse und dem Zwischenstück ein in Richtung intrakorporal geschlossener Ringraum ausgebildet ist, in den die extrakorporale Koppelungseinrichtung setzbar ist, und darüber hinaus einen auf die Außenwandung der Buchse aufgebrachten Schlauch aus flexiblem Material und auf den flexiblen Schlauch aufgebrachte metallische Wolle aufweist.

Gegenüber dem bekannten Lager ist die Buchse vorliegend ein starres Element, das nicht etwa durch Injektionskanülen durchdrungen werden kann. Der auf der Außenwandung der Buchse aufgebrachte Schlauch aus flexiblem Material, vorzugsweise aus Silikon, bewirkt einen Ausgleich zwischen dem Gewebe und den Muskeln auf der einen Seite und der starren Verbindung mit dem Knochenstumpf. Die auf den flexiblen Schlauch aufgebrachte metallische Wolle dient zum Eingranulieren von umgebendem Gewebe, welches so die Keimschranke noch deutlich erhöht.

Die Buchse kann durch Aufschrumpfen auf das Zwischenstück fest mit diesem verbunden sein. Alternativ kann sie mit diesem verschweißt sein. Es ist auch möglich, die Buchse und das Zwischenstück einstückig auszubilden.

Die metallische Wolle besteht vorzugsweise aus Titanfasern, einem körperverträglichen Metall. Das Zwischenstück ist vorzugsweise als Doppelkonus mit einem zylindrischen Mittelabschnitt ausgebildet, mit dem die Buchse verbunden ist.

Keime oder Schmutzpartikel können beim Einsatz des erfindungsgemäßen Lagers nicht zum Knochenstumpf vordringen, sondern werden wirksam abgeblockt. Ein versehentliches Eindringen von beispielsweise Keimen durch Anwendung einer Injektionsnadel ist nicht möglich, da die Buchse aus einem starren, vorzugsweise metallischen Material besteht. Besonders bevorzugt wird Titan als Werkstoff.

Die Keimschranke kann gemäß einer besonders bevorzugten Weiterbildung noch weiter erhöht werden dadurch, dass auf den flexiblen Schlauch ein mehrlagiges metallisches Gewirke und/oder Gewebe und/oder Geflecht und/oder eine mehrlagige metallische Wolle aufgebracht ist, welche(s) sich kappenförmig um das Zwischenstück herum erstreckt. Gegenüber dem Lager gemäß dem Hauptanspruch wird vorliegend also ein mehrlagiges metallisches Gewirke, Gewebe, Geflecht und/oder eine mehrlagige metallische Wolle verwendet, in welche(s) Gewebematerial eingranulieren kann und somit eine noch weiter erhöhte Keimschranke bietet. Das distalseitige Material des flexiblen Schlauches, welches sich von der Buchse kappenförmig erstreckt, ist sehr viel kürzer als das Gitternetzwerk. Vorliegend ist die Fläche, die von dem mehrlagigen metallischen Gewirke, Gewebe, Geflecht und/oder der mehrlagigen metallischen Wolle eingenommen wird, sehr viel größer als bei dem Lager gemäß Hauptanspruch.

Der flexible Schlauch und die sich distalseitig anschließende kappenförmige Verlängerung dienen der Isoelastizität und ermöglichen Mikrobewegungen. Der flexible Schlauch besteht wieder vorzugsweise aus Silikon.

Damit sich das kappenförmig um das Zwischenstück herum erstreckende mehrlagige metallische Gewirke, Gewebe, Geflecht und/oder die mehrlagige metallische Wolle, leichter handhaben lässt, also während der Operation leichter den anatomischen Gegebenheiten anzupassen ist, ist gemäß einer besonders bevorzugten Ausführungsform vorgesehen, dass das mehrlagige metallische Gewirke, Gewebe, Geflecht und/oder die mehrlagige metallische Wolle vorzugsweise am Rand radial verlaufende Linien aufweist, entlang derer die mehreren Lagen miteinander verschweißt sind. Diese Verschweißungen werden vorzugsweise mittels eines Lasers erzeugt. Diese Linien ermöglichen eine bessere Anpassbarkeit an die anatomischen Gegebenheiten. Es ist auch denkbar, diese Linien als Schnittlinien anzusehen, entlang derer das mehrlagige metallische Gewirke, Gewebe, Geflecht und/oder die mehrlagige metallische Wolle auf Größe zugeschnitten werden kann, wobei aufgrund der Verschweißungen ein Ausfransen des mehrlagigen metallischen Gewirkes, Gewebes, Geflechtes und/oder der mehrlagigen metallischen Wolle nicht stattfindet.

Eine weitere bevorzugte Ausführungsform sieht darüber hinaus wengistens zwei geschlitzte, abgewinkelte Klemmringe vor, die über die Buchse setzbar sind, derart, dass sie mit geringem Spiel intraoperativ auf der Buchse längsverschieblich sind und zwischen sich jeweils mindestens eine Lage eines metallischen Gewirkes, Gewebes, Geflechtes und/oder aufgebrachter metallischer Wolle einfassen, welche sich kappenförmig um das Zwischenstück herum erstreckt.

Mit dieser besonders bevorzugten Ausführungsform wird erreicht, dass das Implantat einen möglichst spannungsfreien Verbund mit dem umgebenden Gewebe eingehen kann. Dadurch, dass die wenigstens beiden Klemmringe mit wenigstens der einen Lage zwischen sich längsverschieblich auf der Buchse gelagert sind, ist die Position der Lage dem Operationsumfeld entsprechend einzustellen, und zwar vollkommen abgekoppelt von der Position des flexiblen Schlauches auf der Außenwand der Buchse sowie der daran aufgebrachten metallischen Wolle. Die Ausführungsform verleiht dem Operateur mehr Freiheitsgrade als die vorstehend erwähnte Ausführungsform, bei der der flexible Schlauch im distalen Bereich übergeht zu einer kappenartigen Einfassung des Zwischenstückes. Bei der hier beschriebenen vorteilhaften Ausführungsform ist der flexible Schlauch auf der Buchse erheblich kürzer ausgebildet, um die Einstellmöglichkeit hinsichtlich der Position der Lage, in welche Bindegewebe eingranulieren soll, darzustellen. Wesentlich hierbei ist also die Entkoppelung der Lage beispielsweise aus metallischer Wolle, welche das Zwischenstück umgibt, von der metallischen Wolle, welche mit dem Silikon-Schlauch auf der Buchse verbunden ist. Allein hierdurch ergibt sich die Positionseinstellmöglichkeit. Ein weitgehend spannungsfreier Verbund zwischen dem Implantat und dem umgebenden Gewebe wird somit ermöglicht und somit ein dauerhaftes Einheilen des Implantates im Gliedmaßenstumpf.

Diese Ausführungsform wird vorteilhaft noch dadurch weitergebildet, dass bei mehr als zwei Klemmringen zwischen jeweils benachbarten Klemmringen die genannten Lagen angeordnet sind. Abhängig von den räumlichen Gegebenheiten in situ kann es angezeigt sein, mehr als zwei Klemmringe, also beispielsweise drei oder vier Klemmringe auf die Buchse zu schieben, wobei eben zwischen jeweils zwei Klemmringen stets eine Lage aus beispielsweise der metallischen Wolle angeordnet ist. In diesem Falle werden mehrere Lagen von beispielsweise metallischer Wolle eingebracht, wodurch die Keimschranke nochmals erhöht wird.

Die vorgenannte Ausführungsform kann noch weiter vorteilhaft weitergebildet werden durch einen geschlitzten, abgewinkelten Abschlussring, der mit geringem Spiel an die Innenwandung des Ringraumes greift und die distale Lage der beispielsweise metallischen Wolle gegen den nächsten Klemmring arretiert. Dieser Abschlussring wird erst dann eingesetzt, wenn der Operateur die optimale Anzahl von Klemmringen und Lagen aus beispielsweise metallischer Wolle ermittelt hat und die aufgebaute Anordnung dann abschließen will. Nach Eindrücken des Abschlussringes in die Buchse ist eine Längsverschiebung der Anordnung auf der Buchse nicht mehr möglich.

Die Erfindung wird anhand einiger Ausführungsbeispiele gemäß den Zeichnungsfiguren näher erläutert.

Hierbei zeigt:

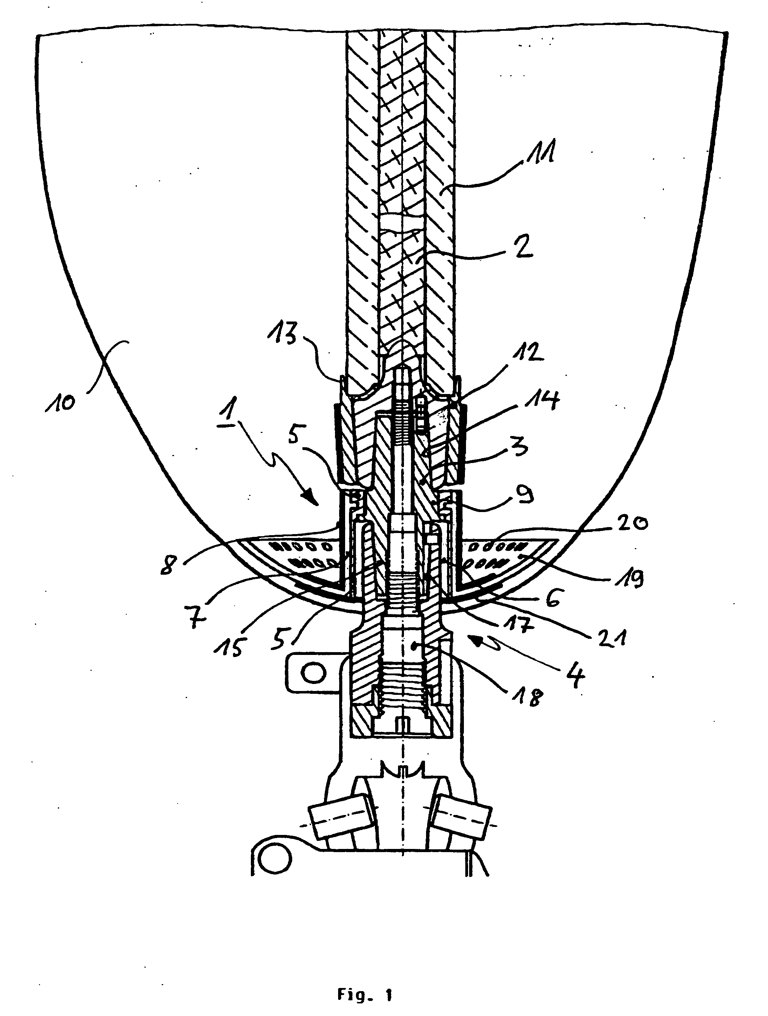

- Fig. 1: einen schematischen Schnitt durch einen Oberschenkelstumpf und durch das implantierte subkutane Lager gemäß einer ersten Ausführungsform,
- Fig.2: schematisch das Zwischenstück des Lagers mit der kappenförmigen Einfassung durch die mehreren Lagen des metallischen Gewirkes, Gewebes, Geflechtes und/oder der metallischen Wolle, teilweise im Schnitt, gemäß einer bevorzugten Weiterbildung,
- Fig. 3: die Aufsicht, von distal gesehen, auf die kappenförmige Einfassung gemäß Fig. 2. und
- Fig. 4: schematisch das Zwischenstück des Lagers einer weiteren Ausführungsform.

Nachfolgend sind gleiche Teile mit demselben Bezugszeichen versehen.

Der Oberschenkelstumpf ist allgemein mit dem Bezugszeichen 10 versehen. Bei dem Knochenstumpf 11 handelt es sich vorliegend um einen Femurstumpf. In den Femurstumpf 11 ist eine starres transkutanes Implantat 2 gesetzt. Es ist distalseitig abgeschlossen durch eine Metallhülse 12, die proximal einen umlaufenden Flansch 13 aufweist, der den Femurstumpf 11 einfasst.

Im Inneren der Metallhülse 12 ist eine konische Klemmhülse 14 ausgebildet. Diese ist vorgesehen zur Herstellung einer konischen Klemmverbindung mit dem vorliegend als Doppelkonus ausgebildeten Zwischenstück 3. Das Zwischenstück 3 weist einen zylindrischen Mittelabschnitt 9 auf, auf dem die Buchse 5 vorliegend aufgeschrumpft ist. Dem Mittelabschnitt 9 schließt sich distalseitig ein weiterer Konus 15 zur Herstellung einer konischen Verklemmung mit einer konischen Klemmhülse 17 in einem Adapter der extrakorporalen Kopplungseinrichtung 4 an.

Die Buchse 5 ist so ausgebildet, dass zwischen ihrer Wandung und dem Zwischenstück ein in Richtung intrakorporal oder proximal geschlossener Ringraum 6 ausgebildet ist. In diesen Ringraum 6 ist die extrakorporale Kopplungseinrichtung 4 gesetzt. Mit einem Sicherungsmittel 18 wird die Verbindung zwischen dem subkutanen Lager und der Koppelungseinrichtung 4 arretiert.

Auf die Außenwandung der Buchse 5 ist ein Schlauch 7 aus Silikon aufgebracht. Der Schlauch 7 kann auf die Buchse 5 aufgeschrumpft und/oder dort verklebt sein. Der Schlauch 7 gestattet Ausgleichsbewegungen des starren Implantates gegenüber dem es umgebenden Gewebe. Mit dem flexiblen Schlauch 7 ist eine Schicht von metallischer Wolle 8 verklebt. Die metallische Wolle dient als Keimschranke nach Eingranulieren von umgebendem Gewebe.

Distalseitig geht der flexible Schlauch 7 einstückig über in ein flexibles Gitternetzwerk 19 mit einer großen Anzahl von Durchbrechungen 20, welche nach der Operation im Laufe der Zeit durchsetzt werden von muskulärem Gewebe.

Noch weiter distalseitig ist mit dem Gitternetzwerk 19 metallische Wolle 21 verklebt, welche nach Eingranulieren von Gewebematerial als weitere Keimschranke dient.

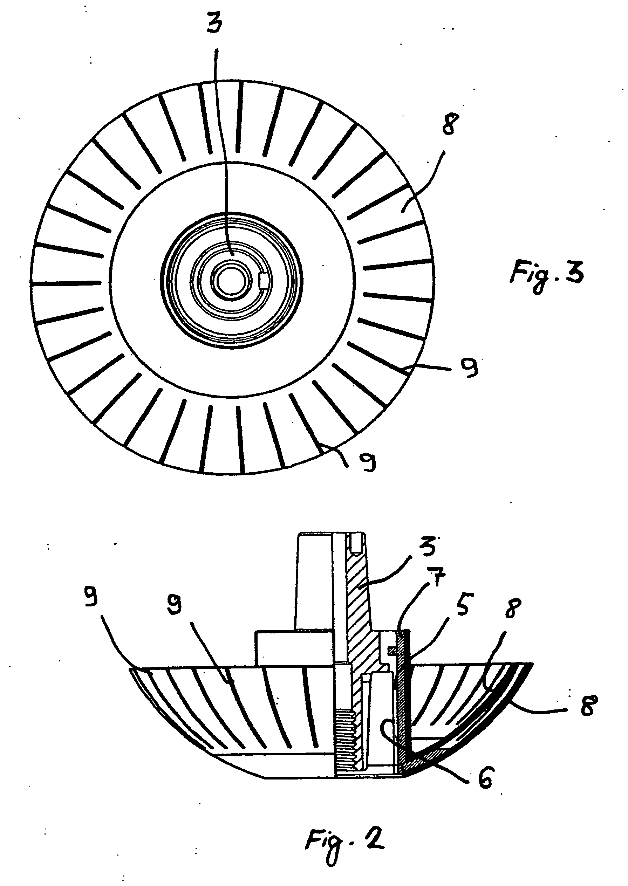

Bei der bevorzugten Weiterbildung gemäß den Figuren 2 und 3 ist mit dem Zwischenstück 3 wieder eine starre Buchse 5 fest verbunden. Die Anordnung ist wiederum so, dass zwischen der Wandung der Buchse 5 und dem Zwischenstück 3 ein in Richtung intrakorporal geschlossener Ringraum 6 ausgebildet ist, in welchen die extrakorporale Kopplungseinrichtung setzbar ist.

Auf die Außenwandung der Buchse 5 ist der Schlauch 7 aus flexiblem Material aufgebracht, distalseitig ist dieser flexible Schlauch 7 so verlängert, dass er die Buchse 5 kappenförmig umfasst. Das flexible Material des Schlauches 7 verleiht der Anordnung die gewünschte Isoelastizität.

Der Schlauch 7 ist außen mit einer Lage eines mehrlagigen metallischen Gewirkes, Gewebes, Geflechtes und/oder einer mehrlagigen metallischen Wolle bedeckt. Eine weitere Lage des mehrlagigen metallischen Gewirkes, Gewebes, Geflechts und/oder der mehrlagigen metallischen Wolle 8 ist distalseitig angeordnet und zwar so, dass eine Lage auf jeder Seite des umgebogenen Endes des Schlauches 7 liegt. Von beiden Seiten kann also Gewebematerial eingranulieren, so dass das mehrlagige metallische Gewirke, Gewebe, Geflecht und/oder die mehrlagige metallische Wolle eine sichere Keimschranke ausbildet.

Damit die mehreren Lagen von metallischem Gewirke, Gewebe, Geflecht und/oder metallischer Wolle flexibel den anatomischen Gegebenheiten angepasst werden können, sind vorliegend radial verlaufende Linien 9 vorgesehen, entlang derer die mehreren Lagen des metallischen Gewirkes, Gewebes, Geflechtes und/oder der metallischen Wolle miteinander verschweißt sind. Sollen die Linien 9 als Schnittmuster dienen, so führt die Verschweißung dazu, dass ein Ausfransen des mehrlagigen metallischen Gewirkes, Gewebes, Geflechts und/oder der mehrlagigen metallischen Wolle nicht stattfindet.

Fig. 2 zeigt die Ansicht von distal und veranschaulicht noch einmal die Anordnung der Linien 9.

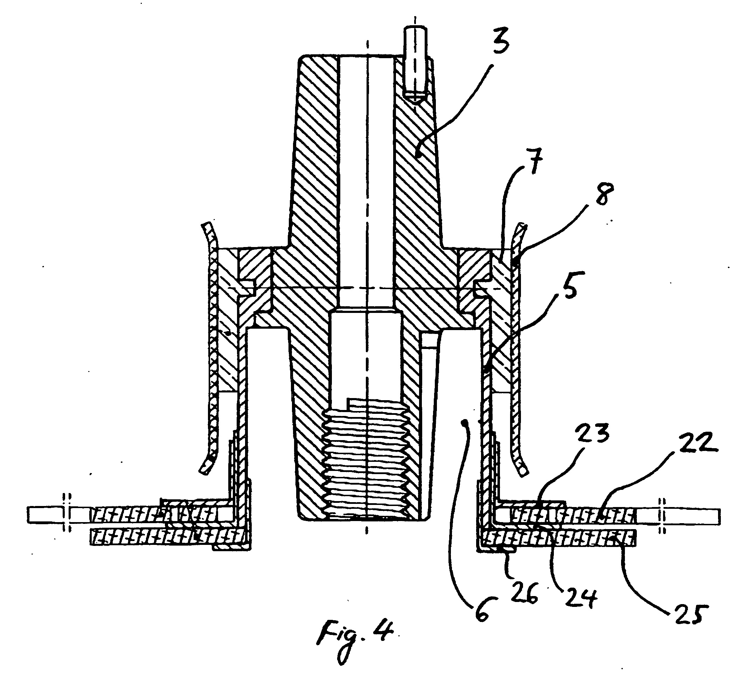

Eine noch weitere Ausführungsform zeigt Fig. 4. Hier ist der auf der Buchse 5 sitzende flexible Schlauch 7 mit der darauf aufgebrachten metallischen Wolle 8 vollkommen entkoppelt von den distalen Massnahmen zum Aufbau einer Keimschranke. Auf die Buchse 5 sind vorliegend zwei geschlitzte Klemmringe 23 und 24 geschoben, die so abgewinkelt sind, dass ein Schenkel parallel zur Wandung der Buchse 5 verläuft und der jeweils andere Schenkel von der Buchse abragt. Dies erlaubt die partielle Einfassung einer ersten Lage 22 eines metallischen Gewirkes, Geflechtes und/oder metallischer Wolle, in welche(s) nach der Operation das umgebende Gewebe eingranulieren kann und so eine Keimschranke aufbaut.

In dem dargestellten Ausführungsbeispiel ist zwischen dem distalen Klemmring 24 und einem geschlitzten und abgewinkelten Abschlussring 26 noch eine weitere Lage 25 eines metallischen Gewirkes, Gewebes, Geflechtes und/oder aufgebrachter metallischer Wolle angeordnet. Der Abschlussring 26 weist einen Schenkel auf, der parallel zur Innenwandung der Buchse 5 verläuft, während der andere Schenkel frei von der Buchse abragt. Während der Operation wird der Operateur die Klemmringe 23 und 24 den örtlichen Gegebenheiten auf der Buchse 5 entsprechend solange verschieben, bis eine weitgehend spannungsfreie Position des Implantates in dem das Implantat später umgebenden Gewebe zu erwarten ist. Ist diese Position gefunden, wird diese durch das Einbringen des einen Schenkels das Abschlussrings 26 in den Ringraum arretiert.

Die Lagen 22 und 25 können bevorzugt so ausgebildet sein wie die metallische Wolle 8 in der weiter oben erläuterten Ausführungsform und wie in Fig. 3 dargestellt.

## Patentansprüche

1. Subkutanes, intramuskuläres Lager (1) für ein starres transkutanes Implantat (2), welches intrakorporal in einem Knochenstumpf verankerbar ist und welches ein Zwischenstück (3) zwischen dem intrakorporal zu verankernden Teil (2) und einer daran ankoppelbaren extrakorporalen Kopplungseinrichtung (4) aufweist,
**gekennzeichnet durch**,
- eine mit dem Zwischenstück (3) fest verbundene starre Buchse (5) derart, dass zwischen der Wandung der Buchse (5) und dem Zwischenstück (3) ein in Richtung intrakorporal geschlossener Ringraum (6) ausgebildet ist, in den die extrakorporale Kopplungseinrichtung (4) setzbar ist,
- einen auf die Außenwandung der Buchse (5) aufgebrachten Schlauch (7) aus flexiblem Material, und
- auf den flexiblen Schlauch (7) aufgebrachte metallische Wolle (8).

2. Lager nach Anspruch 1, **dadurch gekennzeichnet, dass** die Buchse (5) durch Aufschrumpfen auf das Zwischenstück (3) fest mit diesem verbunden ist.

3. Lager nach Anspruch 1, **dadurch gekennzeichnet, dass** die Buchse (5) mit dem Zwischenstück (3) verschweißt ist.

4. Lager nach Anspruch 1, **dadurch gekennzeichnet, dass** die Buchse (5) mit dem Zwischenstück (3) einstückig ausgebildet ist.

5. Lager nach einem der Ansprüche 1 bis 4, **dadurch gekennzeichnet, dass** der flexible Schlauch (7) aus Silikon besteht.

6. Lager nach einem der Ansprüche 1 bis 5, **dadurch gekennzeichnet, dass** die metallische Wolle (8) aus Titanfasern gebildet ist.

7. Lager nach einem der Ansprüche 1 bis 6, **dadurch gekennzeichnet, dass** das Zwischenstück (3) ein Doppelkonus mit einem zylindrischen Mittelabschnitt (9) ist, mit welchem die Buchse (5) verbunden ist.

8. Lager nach einem der Ansprüche 1 bis 7, **gekennzeichnet durch** auf den flexiblen Schlauch (7) aufgebrachtes mehrlagiges metallisches Gewirke, Gewebe, Geflecht und/oder aufgebrachte mehrlagige metallische Wolle (8), welche(s) sich kappenförmig um das Zwischenstück (3) herum erstreckt.

9. Lager nach Anspruch 8, **dadurch gekennzeichnet, dass** sich der flexible Schlauch (7) von der Buchse (5) kappenförmig erstreckt.

10. Lager nach Anspruch 9, **dadurch gekennzeichnet, dass** der flexible Schlauch (7) beidseitig mit mehrlagigem metallischen Gewirke, Gewebe, Geflecht und/oder einer mehrlagigen metallische Wolle (8) belegt ist.

11. Lager nach einem der Ansprüche 8 bis 10, **dadurch gekennzeichnet, dass** das mehrlagige metallisches Gewirke, Gewebe, Geflecht und/oder die mehrlagige metallische Wolle (8) radial verlaufende Linien (9) aufweist, entlang derer die mehreren Lagen miteinander verschweißt sind.

12. Lager nach einem der Ansprüche 1 bis 7, **gekennzeichnet durch** wenigstens zwei geschlitzte, abgewinkelte Klemmringe (23, 24), die über die Buchse (5) setzbar sind, derart, dass sie mit geringem Spiel intraoperativ auf der Buchse (5) längsverschieblich sind und zwischen sich jeweils eine Lage (22, 25) eines metallischen Gewirkes, Gewebes, Geflechtes und/oder aufgebrachter metallischer Wolle partiell einfassen, welche sich kappenförmig um das Zwischenstück (3) herum erstreckt.

13. Lager nach Anspruch 12, **dadurch gekennzeichnet, dass** bei mehr als zwei Klemmringen zwischen jeweils benachbarten Klemmringen die genannten Lagen angeordnet sind.

14. Lager nach Anspruch 12 oder 13, **gekennzeichnet durch** einen geschlitzten, abgewinkelten Abschlussring (26), der mit geringem Spiel an der Innenwandung des Ringraumes (6) anliegt und die distale Lage (25) gegen den nächsten Klemmring (24) arretiert.

## Claims

1. Subcutaneous, intramuscular bearing (1) for a rigid transcutaneous implant (2), which can be anchored intracorporeally in a bone stump and which has an intermediate piece (3) between the part (2) to be anchored intracorporeally and an extracorporeal coupling device (4) which can be coupled thereto,
**characterised by**
- a rigid bush (5) firmly connected to the intermediate piece (3) such that an annular gap (6) closed in the intracorporeal direction, into which the extracorporeal coupling device (4) can be placed, is formed between the wall of the bush (5) and the intermediate piece (3),
- a hose (7) made from flexible material attached to the outer wall of the bush (5), and
- metallic wool (8) attached to the flexible hose (7).

2. Bearing according to claim 1, **characterised in that** the bush (5) by shrinking onto the intermediate piece (3) is firmly connected to the latter.

3. Bearing according to claim 1, **characterised in that** the bush (5) is welded to the intermediate piece (3).

4. Bearing according to claim 1, **characterised in that** the bush (5) is designed to be integral with the intermediate piece (3).

5. Bearing according to one of claims 1 to 4, **characterised in that** the flexible hose (7) consists of silicone.

6. Bearing according to one of claims 1 to 5, **characterised in that** the metallic wool (8) is formed from titanium fibres.

7. Bearing according to one of claims 1 to 6, **characterised in that** the intermediate piece (3) is a double cone with a cylindrical central section (9), to which the bush (5) is connected.

8. Bearing according to one of claims 1 to 7, **characterised by** multi-layer metallic knitted fabric, textile fabric, braid attached to the flexible hose (7) and/or attached multi-layer metallic wool (8), which extends like a cap around the intermediate piece (3).

9. Bearing according to claim 8, **characterised in that** the flexible hose (7) extends like a cap from the bush (5).

10. Bearing according to claim 9, **characterised in that** the flexible hose (7) is covered on both sides with multi-layer metallic knitted fabric, textile fabric, braid and/or a multi-layer metallic wool (8).

11. Bearing according to one of claims 8 to 10, **characterised in that** the multi-layer metallic knitted fabric, textile fabric, braid and/or the multi-layer metallic wool (8) has radially running lines (9), along which the several layers are welded to one another.

12. Bearing according to one of claims 1 to 7, **characterised by** at least two slotted, angular clamping rings (23, 24), which can be placed over the bush (5), such that they are longitudinally displaceable on the bush (5) intraoperatively with slight clearance and between them in each case partially enclose a layer (22, 25) of a metallic knitted fabric, textile fabric, braid and/or attached metallic wool, which extends like a cap around the intermediate piece (3).

13. Bearing according to claim 12, **characterised in that** in the case of more than two clamping rings, the said layers are arranged between in each case adjacent clamping rings.

14. Bearing according to claim 12 or 13, **characterised by** a slotted, angular closure ring (26), which rests with slight clearance against the inner wall of the annular gap (6) and locks the distal layer (25) against the next clamping ring (24).

## Revendications

1. Palier (1) sous-cutané intramusculaire pour un implant (2) transcutané rigide, lequel peut être ancré sous forme intracorporelle dans un moignon osseux et lequel comporte une partie intermédiaire (3) entre la partie (2) à ancrer en intracorporel et un dispositif de couplage (4) extracorporel à coupler à cette dernière,
**caractérisé par**
- un coussinet (5) rigide fermement relié à la pièce intermédiaire (3) de telle sorte qu'il se forme, entre la paroi du coussinet (5) et la pièce intermédiaire (3), un espace annulaire (6) fermé dans la direction intracorporelle, dans lequel peut être mis en place le dispositif de couplage (4) extracorporel,
- un tuyau (7) en matériau flexible appliqué sur la paroi extérieure du coussinet (5), et
- une laine métallique (8) appliquée sur le tuyau flexible (7).

2. Palier selon la revendication 1, **caractérisé en ce que** le coussinet (5) est relié fermement à la pièce intermédiaire (3) par frettage.

3. Palier selon la revendication 1, **caractérisé en ce que** le coussinet (5) est soudé à la pièce intermédiaire (3).

4. Palier selon la revendication 1, **caractérisé en ce que** le coussinet (5) est réalisé d'un seul tenant avec la pièce intermédiaire (3).

5. Palier selon l'une quelconque des revendications 1 à 4, **caractérisé en ce que** le tuyau flexible (7) est réalisé en silicone.

6. Palier selon l'une quelconque des revendications 1 à 5, **caractérisé en ce que** la laine métallique (8) est formée de fibres de titane.

7. Palier selon l'une quelconque des revendications 1 à 6, **caractérisé en ce que** la pièce intermédiaire (3) est un double cône avec une partie centrale (9) cylindrique, à laquelle est assemblé le coussinet (5).

8. Palier selon l'une quelconque des revendications 1 à 7, **caractérisé par** un maillage, tissage, entrelacement métalliques multicouches appliqué sur le tuyau flexible (7) et/ou une laine métallique (8) multicouche appliquée sur ledit tuyau, lesquels/laquelle enserrent la pièce intermédiaire (3) à la manière d'un capuchon.

9. Palier selon la revendication 8, **caractérisé en ce que** le tuyau flexible (7) s'étend en forme de capuchon à partir du coussinet (5).

10. Palier selon la revendication 9, **caractérisé en ce que** le tuyau flexible (7) est revêtu sur les deux faces par un maillage, tissage, entrelacement métalliques multicouches et/ou une laine métallique (8) multicouche.

11. Palier selon l'une quelconque des revendications 8 à 10, **caractérisé en ce que** le maillage, tissage, entrelacement métalliques multicouches et/ou la laine métallique (8) multicouche comportent des lignes (9) orientées radialement, le long desquelles les différentes couches sont soudées entre elles.

12. Palier selon l'une quelconque des revendications 1 à 7, **caractérisé par** au moins deux bagues de serrage (23, 24) coudées et fendues, qui peuvent être posées sur le coussinet (5), de telle sorte qu'en peropératoire elles sont mobiles longitudinalement sur le coussinet (5) avec un faible jeu et reçoivent partiellement entre elles respectivement une couche (22, 25) d'un maillage, tissage, entrelacement métalliques et/ou d'une laine métallique appliquée, qui enserre la pièce intermédiaire (3) à la manière d'un capuchon.

13. Palier selon la revendication 12, **caractérisé en ce que**, en présence de plus de deux bagues de serrage, lesdites couches sont disposées entre respectivement deux bagues de serrage adjacentes.

14. Palier selon la revendication 12 ou 13, **caractérisé par** une bague d'extrémité (26) coudée et fendue, qui prend appui avec un faible jeu sur la paroi intérieure de l'espace annulaire (6) et bloque la couche distale (25) contre la bague de serrage (24) suivante.
